# EUROPEAN PATENT APPLICATION

(11) **EP 4 555 979 A1**
(43) Date of publication of application: **21.05.2025**
(21) Application number: 23210370.5
(22) Date of filing: 16.11.2023
(51) Int. Cl.: A61F 5/01

(54) **FOOT PLATE FOR AFO KIT, METHOD AND USE THEREOF**

(71) Applicant: Comfil ApS, 8883 Gjern (DK)
(72) Inventor: Bak, Henning, 8883 Gjern (DK)
(74) Representative: Patrade A/S

(57) **Abstract**

Foot plate (10) for a jointed ankle-foot orthosis (AFO) (100) comprising an ankle joint (30) with a first arm (32) and a second arm (34) pivotably joined at a pivot point (36), said foot plate comprising at least one aperture (12) adapted to receive the first arm and where the foot plate is adapted to be releasably mounted to the ankle joint by fastening means (20) interlocking the first arm in the aperture in a final position (40) defined by a distance (d) between the foot plate and the pivot point.

## Description

### Field of the Invention

The present invention relates to jointed ankle-foot orthoses (AFO) comprising an ankle joint with a first arm and a second arm pivotably joined at a pivot point.

The invention relates to a foot plate, an ankle joint, a coupling between the foot plate and the ankle joint and an upright respectively for a jointed AFO.

The invention furthermore relates to a method for obtaining such parts and the combination of such parts for a jointed AFO. The invention provides for achieving a modular jointed AFO, a kit for such a modular jointed AFO and a height-adjustable jointed AFO.

### Background of the Invention

Jointed ankle-foot orthoses (AFOs) are braces that structurally compensate for weak or deformed ankle joints. Jointed AFOs are typically used for limiting the range of motion of an ankle to prevent the foot from dropping or rotating into an undesirable position.

Today there exists a large number of alternative jointed AFOs differing in the specific character of the ankle joint, of the foot plate, of the upright or the calf band. However, a general feature of the jointed AFOs is that it is made as one assembled and customized device requiring highly specialized methods for obtaining the customized device.

Obtaining a customized jointed AFO can be a complicated and time-consuming process for the patient and all the elements have to be precisely adjusted during the process of making the AFO, where any adjustments or replacement of elements to the AFO afterwards are rather complicated.

Another limitation to customizing AFOs is that the ankle joints are typically produced in standard lengths and thus the height dimension relating to the distance between the heel and actual ankle joint axis of the patient must be precisely incorporated in the foot plate, which complicates the process of obtaining the foot plate.

### Object of the Invention

One objective of the present disclosure is to achieve an alternative to the prior art.

It may be seen as an object of the present invention to provide parts, methods for obtaining such parts and a combination of parts for a jointed ankle-foot orthosis (AFO) that solves the above-mentioned problems.

Further objectives include providing a modular jointed AFO, a kit for such a modular jointed AFO and a height-adjustable jointed AFO.

### Description of the Invention

One objective of the invention is achieved by a foot plate for a jointed ankle-foot orthosis (AFO) comprising an ankle joint with a first arm and a second arm pivotably joined at a pivot point.

The foot plate comprises at least one aperture adapted to receive the first arm. The foot plate is adapted to be releasably mounted to the ankle joint by fastening means interlocking the first arm in the aperture in a final position. The final position is defined by a distance, d between the foot plate and the pivot point.

The distance is measured from a specified point on the foot plate for example from the lower heel rest.

One effect of the foot plate being releasably mounted to the first joint arm is that the foot plate can be retrofitted to an existing jointed AFO or vice versa, the foot plate can be reused with a new ankle joint in case of wearing out, uneven wear or simply the need for a new, more functional ankle joint or a joint with other characteristics.

The foot plate may be configured with two apertures, each adapted to receive the first arm of an associated ankle joint, thus achieving a foot plate for a double jointed AFO with a joint on both the inner and outer side of the ankle.

In one embodiment of the foot plate, the aperture is configured to interlock with the first arm in a set of final positions. Each of the final positions is defined by the distance, d between the foot plate and the pivot point being different from the remaining final position(s).

A set refers to two or more.

This embodiment is particularly, but not exclusively, advantageous for providing a foot plate providing height-adjustable jointed AFOs while maintaining the remaining characteristics of the AFO.

In one embodiment of the foot plate, the fastening means comprises holes and one or more locking pins adapted to interlock with the hole(s). The locking pins may preferably be screws or a locking pin with a screw end. The holes or at least the holes receiving the screw ends may preferably be threaded holes in combination with the screws.

In one aspect, the locking pins may comprise a combination of screw and a retainer ring and/or washer with a throughgoing hole for the screw.

The fastening means may include non-threaded holes in the first arm, threaded holes in the aperture in the foot plate and screws to be inserted through the non-threaded holes and engaging with the threaded holes in the foot plate.

Alternative locking pins may be provided in the foot plate adapted to interact with holes in the first arm and a locking mechanism configured to lock the end of the pins so that the arm and the foot plated is interlocked.

In one embodiment, the foot plate is made of a thermoplastic carbon composite.

Unlike thermosets, thermoplastics do not cure and can be easily softened by heating and sets again with cooling at room temperature.

Different grades of thermoplastics exist. Some grades of thermoplastics retain their structural capabilities above 150°C and short term above 250°C. Thermoplastics are characteristic in being heat-resistant, chemical-resistant, corrosion-resistant, and excellent electrical and thermal insulators.

Thermoplastic carbon composites provide for a strong and light construction, it is thermoformable at relatively low temperatures, while enjoying the increased stiffness and flexibility of carbon composites.

Thermoplastic carbon composites have the further advantage that it does not corrode and the material brakes gradually rather than at a snap, which may be beneficial in view of the intended use.

Using thermoplastic carbon composites may furthermore be advantageous in that conventional heating sources can be used, such as a heat gun or an oven, due to the low heating temperature for forming or contouring the material. Furthermore, the composite can be accommodated to smaller radii bends.

In one embodiment, the aperture forms a channel adapted to receive the first arm, with the channel comprising one or more holes configured for receiving a locking pin, preferably a screw or a locking pin with a screw end.

This embodiment may be advantageous in that the channel may help to guide the arm into correct aligned position such that the fastening means are positioned aligned for easy assembly. The channel may comprise holes in two walls facing with the holes aligned and where the first arm comprises one or more complementary holes such that the arm can be fastened in one final position out of a set of final positions.

In this embodiment, the holes in the one wall of the channel may be non-threaded holes and the holes in the other wall be threaded holes for fastening a screw as one fastening means.

The channel may be an open-ended cavity.

In a further embodiment, the foot plate comprises an insert embedded in the foot plate forming the channel. The channel may be an open-ended cavity. The insert may be a metal insert, e.g. an insert made partly of metal or of a combination of metals.

In one aspect, the insert may comprise two or three elements, e.g. a closed frame and a fastening plate or an open frame and two fastening plates with the fastening plate(s) adapted to be arranged in the frame to form an open-ended cavity for receiving the first arm of the ankle joint.

The fastening plates may be made in metal. In case of threaded fastening means, the part comprising the threaded holes should be adapted in accordance with the requirements for holding the screw in a secure manner.

In one embodiment, the thickness of the fastening plates may be in the order of 1-2 mm.

A further objective of the invention is achieved by a combination comprising the foot plate according to any one of the disclosed embodiments and the ankle joint for a jointed AFO. The ankle joint having a first arm and a second arm pivotably joined at a pivot point. The first arm comprises one or more holes configured for receiving a locking pin, preferably a screw.

The embodiment is particularly, but not exclusively, advantageous for providing a combination of parts to a jointed AFO to obtain an AFO with replaceable parts. This is beneficial in that a worn out joint, being an off-the-shelf product, may be replaced such that the customized parts of the AFO, i.e. the foot plate, the calf band and the uprights can be reused with the new ankle joint. This may also be beneficial if a new, more functional ankle joint or a joint with other characteristics is needed.

In a further embodiment of the combination, the one or more holes in the first arm is an adjustable channel. By adjustable channel is meant a channel formed by multiple overlapping holes arranged on a substantially straight line.

The combination of the foot plate in combination with the ankle joint entails the effects already discussed for the foot plate alone and the interaction of the foot plate and the ankle joint achieving a releasable and height-adjustable foot plate relative to the pivot point of the ankle joint.

A further objective of the invention is achieved by a customizable orthotic upright for a jointed ankle-foot orthosis (AFO) comprising an ankle joint with a first arm and a second arm pivotably joined at a pivot point.

The orthotic upright comprises an elongated bar with a calf band end adapted for mechanical fastening for a calf band and a joint end where the elongated bar extends into a terminal adapted for mechanical fastening to the second arm.

In one aspect, the elongated bar is substantially D- shaped.

In one aspect, the elongated bar has a cross section area in the range of 0.5-3 cm², in the range of 0.8-2 cm², or 1-1.5 cm².

In one aspect, the terminal is substantially squared and pre-formed to a mounting element comprised in the second arm.

Thermoplastic carbon composites provide for a strong and light construction, and it is thermoformable at relatively low temperatures, while enjoying the increased stiffness and flexibility of carbon composites.

Thermoplastic carbon composites have the further advantage that it does not corrode and the material brakes gradually rather than at a snap, which may be beneficial in view of the intended use.

Using thermoplastic carbon composites may furthermore be advantageous in that conventional heating sources can be used, such as a heat gun or an oven, due to the low heating temperature for forming or contouring the material. Furthermore, the composite can be accommodated to smaller radii bends.

The thermoplastic carbon composite may delaminate if directly heated as the fibres can delaminate and hence thereby the strength of the material can be significantly decreased. It is therefore beneficial to avoid any direct heating of the terminal.

This embodiment is particularly, but not exclusively, advantageous for providing a customized orthotic upright with a terminal adapted for retrofitting with an existing ankle joint. Hence, only the elongated bar of the upright is heated and fitted to achieve a customized orthotic upright. This may be beneficial to reduce the work required by the orthotist and hence, reduce the production time for customized AFOs.

A further objective of the invention is achieved by a method of obtaining a customized orthotic upright comprising the acts of:
- providing a model of the calf for the orthotic upright to be customized to including a point of the actual ankle joint axis,
- providing the orthotic upright according to the above disclosed embodiment with the second arm fastened to the terminal and with a forming sleeve covering the elongated bar, and performing the following steps of:
   a) heating the covered part of the upright to a temperature above a deformation temperature,
   b) arranging the heated upright along the calf on the model with the pivot point of the joint arm arranged aligned with the point of the actual ankle joint axis,
   c) releasably fixating the heated upright to the model to contour the upright to the model until cooled to settling temperature of the thermoplastic carbon composite, and
   d) removing the forming sleeve,
   wherein the terminal does not receive any mechanical forming or direct heating.

The forming sleeve provides a protection for the thermoplastic carbon composite during the heating step as the fibres can delaminate when heated and hence thereby greatly decrease the strength of the upright.

The terminal is fastened to the mounting element of the second arm and is not heated. Hence, the shape of the terminal is maintained throughout the method.

This is beneficial in that the method can be performed without covering the terminal by a protective sleeve of any kind. Furthermore, by performing the method with the second arm fastened to the upright, the orthotist can manually handle the upright by holding at the terminal end and furthermore ensure correct adjustment according to the actual ankle joint axis.

With the terminal being fastened to the mounting element of the second arm the thermoplastic carbon is not completely covered and thus it is beneficial to avoid any direct heating of the terminal.

It is advantageous to assemble the upright with the second arm before customizing the upright to ensure that the pivot point of the ankle joint is aligned with the point of the actual ankle joint axis on the model.

In one embodiment, the model is provided with an alignment hole at the point of the actual ankle joint axis. The joint arm is provided with a complementary alignment fixture adapted to be arranged in the alignment hole for holding the pivot point arranged aligned with the point of the actual ankle joint axis during the steps of arranging the heated upright along the calf on the model and fixating the heated upright to the model.

This embodiment is advantageous to ensure alignment of the pivot point of the ankle joint and the point of the actual ankle joint axis on the model, especially when handling heated parts and to ensure a more stable fixation of the upright during the contouring or forming of the elongated bar to the calf.

In one embodiment, the forming sleeve is made of silicone.

Silicone is advantageous in that it is form stable at the temperatures used in the disclosed method. Furthermore, the silicone sleeve has a tight fit to the elongated bar and thus applies a compression force to the elongated bar to reduce the risk of delamination.

In one embodiment of the method, the covered part of the upright (50), i.e. the elongated part, is arranged in heated oil for a duration in the range of 8-20 minutes, 10-18 minutes or 12-15 minutes at an oil temperature in the range of 80-200° Celsius, 100-180° Celsius or 120-150° Celsius.

The combination of the time intervals and the temperatures ensures sufficient heating of the upright bar without exposing the elongated bar to excessive heating or unnecessary prolonging the heating step.

Embodiments from the customizable upright should be seen as applicable to the method, and embodiments from the method should be seen as applicable for the customizable upright, and hence the discussed effects and advantages relates to both the method and the upright.

A further objective of the invention is achieved by a combination comprising the customizable orthotic upright according to the disclosed embodiments and the ankle joint for a jointed AFO having a first arm and a second arm pivotably joined at a pivot point, wherein the first arm is configured for mounting a foot plate, said first arm comprising one or more holes configured for receiving a locking pin, preferably a screw.

In one aspect, the one or more holes in the first arm is an adjustable channel.

A further objective of the invention is achieved by a jointed AFO comprising the combination of the foot plate and the ankle joint and the customized orthotic upright according to the herein disclosed embodiments.

The different embodiments of the foot plate, the ankle joint or the upright may each be combined with any of the other devices. These and other aspects of the invention will be apparent from and elucidated with reference to the embodiments described hereinafter.

The embodiments of the individual parts are particularly, but not exclusively, advantageous in being individual parts ready to be assembled and adjusted to the patient. Furthermore, the concept may be retrofitted with off-the-shelf ankle joints by replacing the first arm with a corresponding first arm configured with fastening holes, e.g. an adjustable channel.

### Description of the Drawing

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example need not have all the aspects or advantages shown. An aspect or an advantage described in conjunction with a particular example is not necessarily limited to that example and can be practiced in any other examples even if not so illustrated, or if not so explicitly described.

Exemplary embodiments of the invention are described in the figures, whereon:
Fig. 1 illustrates one embodiment of a foot plate.
Fig. 2 illustrates one embodiment of an insert for forming a channel in the foot plate.
Fig. 3 illustrates alternative embodiments of the coupling for the foot plate and an ankle joint.
Fig. 4 illustrates one embodiment of an upright and a model for contouring the upright.
Fig. 5 illustrates one embodiment of the method of obtaining the customized orthotic upright.

### Detailed Description of the Invention

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects.

Throughout the specification, when an element is referred to as being "connected" to another element, the element is "directly connected" to the other element, "electrically connected", "fluidic connected" or "communicatively connected" to the other element with one or more intervening elements interposed there between.

The terminology used herein is for the purpose of describing particular examples only and is not intended to be limiting. As used herein, the terms "comprises" "comprising" "includes" and/or "including" when used in this specification specify the presence of stated features, integers, steps, operations, elements, and/or components, but do not preclude the presence or addition of one or more other features, integers, steps, operations, elements, components, and/or groups thereof.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

| No | Item |
|---|---|
| d | distance, foot plate to pivot point |
| 10 | foot plate |
| 12 | aperture |
| 14 | channel |
| 16 | fastening plate |
| 20 | fastening means |
| 30 | ankle joint |
| 32 | first arm |
| 34 | second arm |
| 36 | pivot point |
| 38 | mounting element, second arm |
| 40 | final position |
| 50 | orthotic upright |
| 52 | elongated bar |
| 54 | calf band end |
| 56 | joint end |
| 58 | terminal |
| 60 | model |
| 62 | alignment hole |
| 90 | forming sleeve |
| 92 | alignment fixture |
| 100 | jointed ankle-foot orthosis (AFO) |
| 200 | method |
| 202 | providing |
| 204 | heating |
| 206 | arranging |
| 208 | fixating |

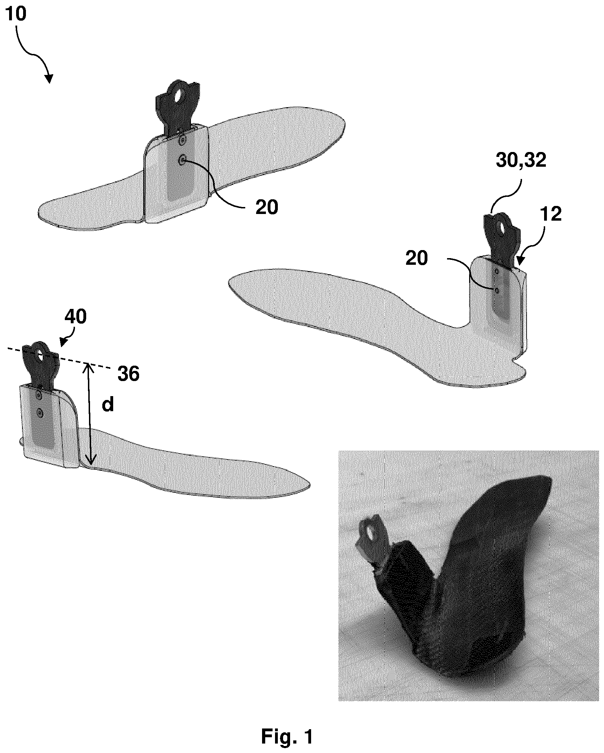

Fig. 1 illustrates one embodiment of a foot plate 10 for a jointed ankle-foot orthosis (AFO). The illustrated foot plate comprises one aperture 12 in the form of a channel adapted to receive the first arm 32 of an ankle joint 30. The foot plate is illustrated with the first arm 32 inserted into the aperture and fastened to the foot plate.

The foot plate is illustrated with an insert embedded in the foot plate to form the aperture.

The foot plate is releasably mounted to the first arm by fastening means 20 interlocking the first arm 32 in the aperture 12 in a final position 40 defined by a distance d between the foot plate 10 and the pivot point 36 of the first arm.

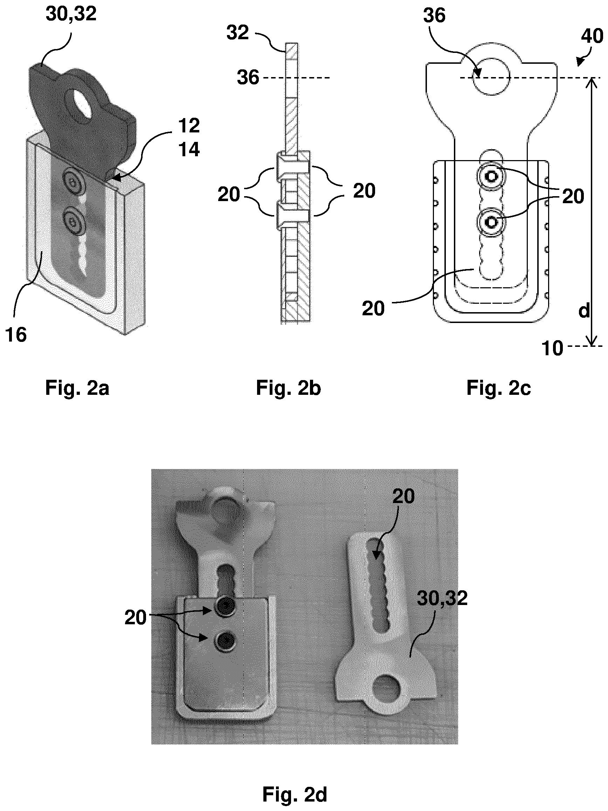

Fig. 2 illustrates one embodiment of a coupling for the foot plate and an ankle joint with one embodiment of an insert for forming a channel 14 in the foot plate. The insert may comprise two elements, one forming an aperture 12 and a fastening plate 16. The fastening plate 16 in this embodiment is adapted to be arranged in the aperture 12 to form an open-ended cavity for receiving the first arm 32 of an ankle joint.

The fastening plate 16 comprises two holes 20 aligned with the two corresponding holes 20 in the element forming the aperture 12 when the fastening plate 16 is arranged in the aperture.

The first arm 32 of the ankle joint comprises one hole 20 in the form of an adjustable channel. Two screws are inserted through the holes in the fastening plate, through the adjustable channel in the first arm and into the two holes in the element forming the aperture. The two holes in the element may be threaded holes adapted for fastening the screws.

The screws and holes in combinations are considered the fastening means 20.

The position of the first arm relative to the insert and hence relative to the foot plate can be adjusted by simply removing the screws displacing the arm in the channel, repositioning the screws and fasten. Thereby achieving the height adjustable AFO, illustrated in fig. 2c with the distance d and the first arm being arranged in final position 40 selected between a set of final positions. The distance d is the distance from the pivot point 36 of the ankle joint and a specified position on the foot plate 10, not illustrated here.

Fig. 2a, 2b and 2c are sketched illustrations of the insert and first arm, whereas fig. 2d is a photo of corresponding prototypes.

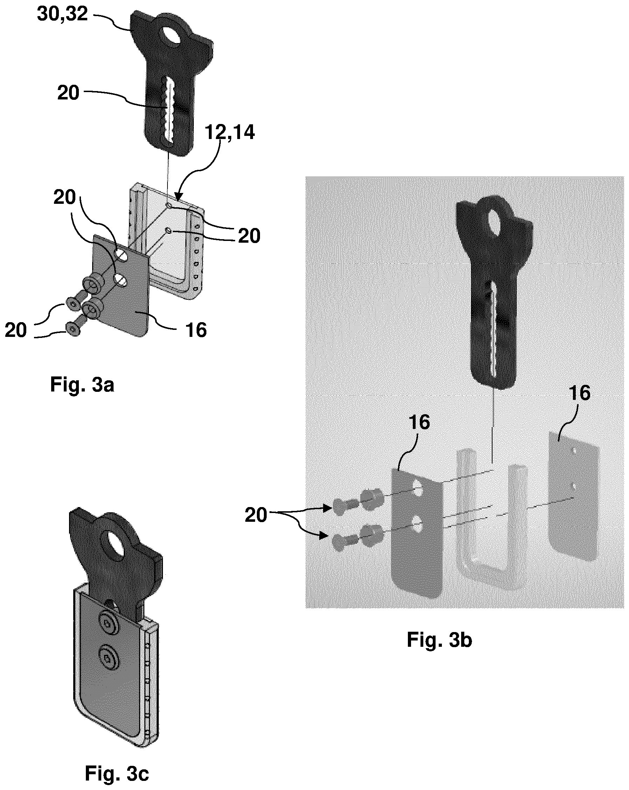

Fig. 3 illustrates an alternative embodiment of the coupling for the foot plate and an ankle joint. The illustrated insert comprises three elements, a frame and two fastening plates 16. The fastening plates 16 are adapted to be arranged in the frame form an open-ended cavity for receiving the first arm 32 of the ankle joint.

The shape of the insert may be provided in a shape in accordance with the form of the foot plate.

The fastening plates may be made in metal. In case of threaded fastening means, the part comprising the threaded holes should be adapted in accordance with the requirements for holding the screw in a secure manner.

In one embodiment, the thickness of the fastening plates may be in the order of 1-2 mm.

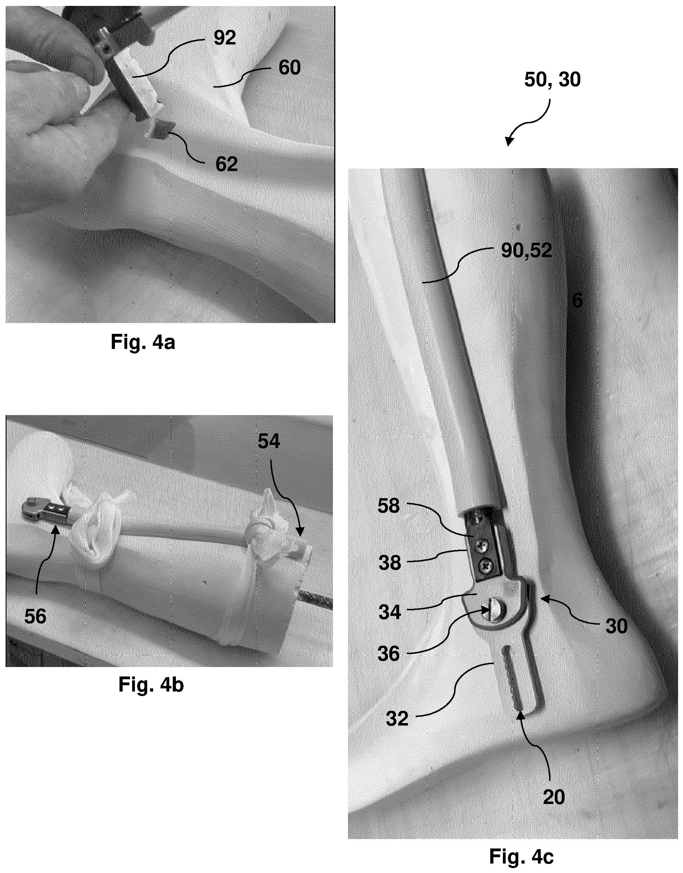

Fig. 4 illustrates one embodiment of a customizable orthotic upright 50 and a model 60 for contouring the upright. Fig. 4 also illustrates embodiments of some of the steps in the method of obtaining a customized orthotic upright 50.

In fig. 4 the upright is illustrated with a forming sleeve 90 covering the part of elongated bar 52 to be heated and subsequently contoured to the modelled calf.

In particular, fig. 4a illustrates one example of how the upright 50 can be aligned with the model 60. The model is provided with an alignment hole 62 at the point of the actual ankle joint axis. The upright is mounted with a second arm of an ankle joint. The second arm comprises the pivot point of the joint and aligned with this pivot point, the second arm is provided with a complementary alignment fixture 92 adapted to be arranged in the alignment hole for holding the pivot point arranged aligned with the point of the actual ankle joint axis. In the illustrated embodiment, the fixture 92 and alignment hole are square-shaped. However, any complementary shapes may be used.

Fig. 4b illustrates one example of how-to releasably fixating the heated upright to the model 60 to contour the upright to the model until cooled to settling temperature. When the upright is heated to a deformation temperature, the upright is arranged along the calf on the model with the pivot point of the joint arm arranged aligned with the point of the actual ankle joint axis, as illustrated in fig. 4b. The upright is releasably fixed to the calf of the model by strapping the elongated bar to the model at two points between the calf band end 54 and the joint end 56 of the upright. Additional strapping points may be used if expedient.

Fig. 4c illustrates a combination of a customized upright 50 with a mounted ankle joint 30 with a first arm 32 configured for a height adjustable AFO.

Fig. 4 also illustrates how the terminal has been pre-fitted to the mounting element 38 comprised in the second arm 34. The terminal is not heated or heat formed in the customization process of contouring the elongated bar. By not heating the terminal, the need for covering the terminal is omitted resulting in a less complicated process.

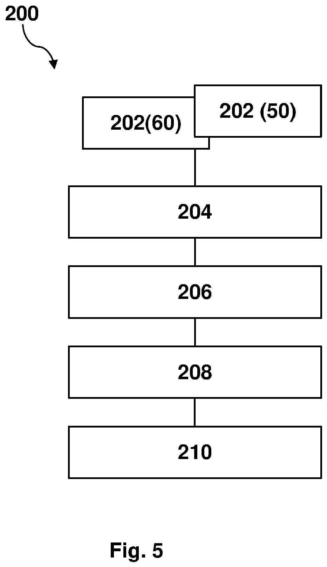

Fig. 5 illustrates one embodiment of the method of obtaining the customized orthotic upright. The method includes the following steps:
- providing 202 a model 60 of the calf for the orthotic upright to be customized to including a point of the actual ankle joint axis,
- providing 202 an orthotic upright 50 with the second arm 34 fastened to the terminal and with a forming sleeve covering the elongated bar and performing the following steps of:
   a) heating 204 the covered part of the upright 50 to a temperature above a deformation temperature,
   b) arranging 206 the heated upright along the calf on the model 60 with the pivot point of the joint arm arranged aligned with the point of the actual ankle joint axis,
   c) releasably fixating 208 the heated upright to the model to contour the upright to the model until cooled to settling temperature of the thermoplastic carbon composite, and
   d) removing 210 the forming sleeve.

## Claims

1. Foot plate (10) for a jointed ankle-foot orthosis (AFO) (100) comprising an ankle joint (30) with a first arm (32) and a second arm (34) pivotably joined at a pivot point (36), said foot plate (10) comprising at least one aperture (12) adapted to receive the first arm (32) and where the foot plate (10) is adapted to be releasably mounted to the ankle joint (30) by fastening means (20) interlocking the first arm (32) in the aperture (12) in a final position (40) defined by a distance (d) between the foot plate (10) and the pivot point (36).

2. The foot plate (10) according to claim 1, wherein the aperture (12) is configured to interlock with the first arm (32) in a set of final positions (40, 40'), each final position (40) defined by the distance (d) between the foot plate and the pivot point different from the remaining final position(s).

3. The foot plate (10) according to any one of claims 1 or 2, wherein the fastening means (40) comprises holes and one or more locking pins adapted to interlock with the hole(s), preferably screws or locking pins with a screw end.

4. The foot plate (10) according to any one of the preceding claims made in thermoplastic carbon composite.

5. The foot plate (10) according to any one of the preceding claims, wherein the aperture (12) forms a channel (14) adapted to receive the first arm (32) and comprising one or more holes configured for receiving a locking pin, preferably a screw or a locking pin with a screw end.

6. The foot plate (10) according to claim 5 comprising an insert, preferably a metal insert embedded in the foot plate forming the channel, preferably an open-ended cavity.

7. Combination comprising the foot plate (10) according to any one of claims 1-6 and the ankle joint (30) for a jointed ankle-foot orthosis (AFO) having a first arm (32) and a second arm (34) pivotably joined at a pivot point (36), wherein the first arm (32) comprises one or more holes configured for receiving a locking pin, preferably a screw.

8. The combination according to claim 7, wherein the one or more holes in the first arm (32) is an adjustable channel.

9. A customizable orthotic upright (50) for a jointed ankle-foot orthosis (AFO) (100) comprising an ankle joint (30) with a first arm (32) and a second arm (34) pivotably joined at a pivot point (36), said orthotic upright (50) comprising an elongated bar (52) with a calf band end (54) adapted for mechanical fastening for a calf band and a joint end (56) where the elongated bare extends into a terminal (58) adapted for mechanical fastening to the second arm (34), wherein said orthotic upright (50) is made in a thermoplastic carbon composite and is customizable to a calf by heating the elongated bar and forming the elongated bar (52) when heated, while the terminal (58) does not receive any mechanical forming or direct heating and where the terminal is preformed to a mounting element (38) comprised in the second arm.

10. A method (200) of obtaining a customized orthotic upright (50) comprising the acts of:
- providing (202) a model (60) of the calf for the orthotic upright to be customized to including a point of the actual ankle joint axis,
- providing (202) the orthotic upright (50) according to claim 9 with the second arm (34) fastened to the terminal (16) and with a forming sleeve (90) covering the elongated bar (52), and performing the following steps of:
a) heating (204) the covered part of the upright (50) to a temperature above a deformation temperature,
b) arranging (206) the heated upright along the calf on the model (60) with the pivot point of the joint arm arranged aligned with the point of the actual ankle joint axis,
c) releasably fixating (208) the heated upright to the model to contour the upright to the model until cooled to settling temperature of the thermoplastic carbon composite, and
d) removing (210) the forming sleeve,
wherein the terminal (58) does not receive any mechanical forming or direct heating.

11. The method (200) according to claim 10, wherein the model is provided with an alignment hole (62) at the point of the actual ankle joint axis, and the joint arm is provided with a complementary alignment fixture (92) adapted to be arranged in the alignment hole for holding the pivot point arranged aligned with the point of the actual ankle joint axis during the steps of arranging (206) the heated upright along the calf on the model and fixating (208) the heated upright to the model.

12. The method (200) according to claims 10 or 11 wherein the forming sleeve (90) is made of silicone.

13. The method (200) according to any one of claims 10-12 wherein the covered part of the upright (50) is arranged in heated oil for a duration in the range of 8-20 minutes, 10-18 minutes or 12-15 minutes at an oil temperature in the range of 80-200° Celsius, 100-180° Celsius or 120-150° Celsius.

14. Combination comprising the orthotic upright (50) according to claim 9 and the ankle joint (30) for a jointed ankle-foot orthosis (AFO) having a first arm (32) and a second arm (34) pivotably joined at a pivot point (36), wherein the first arm (32) is configured for mounting a foot plate, said first arm comprising one or more holes configured for receiving a locking pin, preferably a screw.

15. Jointed ankle-foot orthosis (AFO) (100) comprising the combination of the foot plate (10) and the ankle joint (30) according to claim 7 or 8 and the customized orthotic upright (50) according to claim 9.
